Europäisches Patentamt

European Patent Office

Office européen des brevets

(11) Publication number: **0 122 080**
**B1**

# EUROPEAN PATENT SPECIFICATION

(45) Date of publication of patent specification: **13.09.89**

(21) Application number: **84301995.1**

(22) Date of filing: **23.03.84**

(60) Divisional application **87110601 filed on 22.07.87.**

(51) Int. Cl.⁴: **C 12 N 15/00, C 12 P 21/00 //
C12N9/52, A61K39/395**

(54) A process for the production of a protein.

(30) Priority: **25.03.83 GB 8308234
07.06.83 PCt/gb83/00152
12.10.83 GB 8327345**

(43) Date of publication of application:
**17.10.84 Bulletin 84/42**

(45) Publication of the grant of the patent:
**13.09.89 Bulletin 89/37**

(84) Designated Contracting States:
**AT BE CH DE FR IT LI LU NL SE**

(56) References cited:
**EP-A-0 068 691
WO-A-83/04418
GB-A-1 258 063**

**H.R. MAHLER et al.: "Biological Chemistry,
second edition, 1971, pages 177-183, Harper &
Row Publishers, New York, US;**

The file contains technical information
submitted after the application was filed and
not included in this specification

(73) Proprietor: **CELLTECH LIMITED
244-250 Bath Road
Slough Berkshire SL1 4DY (GB)**

(72) Inventor: **Lowe, Peter Anthony
9, Melrose Avenue
Reading Berkshire (GB)**
Inventor: **Marston, Fiona Angela Olinda
9, Keats Road
Woodley Reading Berkshire (GB)**
Inventor: **Angal, Sarojani
30, Linden Road
Woodley Reading Berkshire (GB)**
Inventor: **Schoemaker, Joyce Ann
16, Fairfax Road
Chiswick London, W4 (GB)**

(74) Representative: **Votier, Sidney David et al
CARPMAELS & RANSFORD 43, Bloomsbury
Square
London WC1A 2RA (GB)**

Courier Press, Leamington Spa, England.

# EP 0 122 080 B1

(56) References cited:
CHEMICAL ABSTRACTS, vol. 87, no. 19, 7th
November 1977, page 216, no. 147755e,
Columbus, Ohio, US; D.M. MULVIHILL et al.:
"Selective denaturation of milk coagulants in 5
M-urea" & J. DAIRY RES. 1977, 44(2), 319-24

CHEMICAL ABSTRACTS, vol. 97, no. 19, 8th
November 1982, page 528, no. 160800s,
Columbus, Ohio, US; AKINORI SUMI et al.:
"Denaturation by guanidine hydrochloride of
Fc(t) and pFc' fragments of human
immunoglobulin G." & J. BIOCHEM. (TOKYO)
1982, 92(3), 823-33

NATURE 285, 1980, 456-61
AMINOSÄUREN, PEPTIDE, PROTEINE, H.D.
Jakubke + H. Jeschkert, Weinheim 1982, pp.
402-404

## Description

This invention relates to the field of protein production using recombinant DNA biotechnology. In particular it relates to a process for the recovery of a protein produced in an insoluble form by a host organism transformed with a vector including a gene coding for the protein.

There are now numerous examples of commercially valuable proteins which may be produced in large quantities by culturing a host organism capable of expressing heterologous genetic material. Once a protein has been produced by a host organism it is usually necessary to treat the host organism in some way, in order to obtain the desired protein. In some cases, such as in the production of interferon in *Escherichia coli* a lysis or permeabilisation treatment alone may be sufficient to afford satisfactory yields. However, some proteins are produced within a host organism in the form of insoluble protein aggregates which are not susceptable to extraction by lysis or permeabilisation treatment alone. It has been reported that a human insulin fusion protein produced in *E. coli* forms insoluble protein aggregates (see D. C. Williams *et al* (1982) Science *215* 687—689).

A protein exists as a chain of amino acids linked by peptide bonds. In the normal bioligically active form of a protein (hereinafter referred to as the native form) the chain is folded into a thermodynamically preferred three dimensional structure, the conformation of which is maintained by relatively weak interatomic forces such as hydrogen bonding, hydrophobic interactions and charge interactions. Covalent bonds between sulphur atoms may form intramolecular disulphide bridges in the polypeptide chain, as well as intermolecular disulphide bridges between separate polypeptide chains of multisubunit proteins, e.g. insulin. The insoluble proteins produced in some instances do not exhibit the functional activity of their natural counterparts and are therefore in general of little use as commercial products. The lack of functional activity may be due to a number of factors but it is likely that such proteins produced by transformed host organisms are formed in a conformation which differs from that of their native form. They may also possess unwanted intermolecular disulphide bonds not required for functional activity of the native protein in addition to intramolecular disulphide bonds. The altered three dimensional structure of such proteins not only leads to insolubility but also diminishes or abolishes the bioligical activity of the protein. It is not possible to predict whether a given protein expresses by a given host organism will be soluble or insoluble.

In our copending British patent application GB 2100737A we describe a process for the production of the proteolytic enzyme chymosin. The process involves cleaving a chymosin precursor protein produced by a host organism which has been transformed with a vector including a gene coding for the relevant protein. In the course of our work we discovered that the chymosin precursor proteins were not produced in their native form but as an insoluble aggregate. In order to produce a chymosin precursor in a native form which may be cleaved to form active native chymosin, the proteins produced by a host organism were solubilised and converted into their native form before the standard techniques of protein purification and cleavage could be applied.

In our copending published International patent application WO 83/04418 the methods used for the solubilisation of chymosin precursor proteins are described. In general the techniques described involved the denaturation of the protein followed by the removal of the denaturant thereby allowing renaturation of the protein. In one example, the denaturant used is a compound such as urea or guanidine hydrochloride. When the insoluble precursor is treated with urea or quanidine hydrochloride it is solubilised. When the denaturant is removed, for example by dialysis, the protein returns to a thermodynamically stable conformation which, in the case of chymosin precursors, is a conformation capable of being converted to active chymosin.

The solubilised protein may be separated from insoluble cellular debris by centrifugation or filtration. The production of proteins from suitably transformed host organisms is potentially of great commercial value. The processes involved are of a type which may be scaled up from a laboratory scale to an industrial scale. However, where the protein produced is formed as an insoluble aggregate, potential complications in the process may increase the cost of production beyond a viable level.

The methods of solubilisation in a strong denaturant such as guanidine hydrochloride or urea described in published British patent application GB2100737A and in published International patent application WO 83/04418 and methods of solubilisation using alkali, each solubilize significant percentages of insoluble proteins found in extracts from host organisms. However, neither is completely quantitative in terms of recovery of native protein. The reasons for this have not been clearly defined and are probably different for the two types of solubilisation. It appears that guanidine hydrochloride solubilises all the material present but only a portion is converted into native proteins after removal of guanidine hydrochloride. Alkali treatment may not allow complete renaturation to form the native form of the protein and in addition does not solubilise all of the insoluble form of the protein. We have discovered that by combining the two methods, a greatly enhanced yield of native protein may be obtained.

Accordingly to the present invention, we provide a process for the production of a soluble native protein in which an insoluble form of the protein is produced by a host organism transformed with a vector including a gene coding for the protein, wherein the insoluble form of the protein is first denatured in an aqueous solution including a denaturant, and subsequently the resulting solution is diluted in an alkaline aqueous solution at a pH selected to promote dissociation of the group or groups of the protein involved in maintaining the conformation of the protein and the protein is renatured by reducing the pH of the solution below a pH effective to denature the protein, to produce the soluble native form of the protein, provided that (for designated states other than Italy) the soluble native protein is not a chymosin precursor.

The dilution introduces an element of physical separation between the denatured molecules, before renaturation is brought about, for example, by neutralisation of the alkaline denaturing solution. The dilution and resulting physical separation of the denatured molecules appears to assist their renaturation in native form. The solubilisation process described immediately above leads to a recovery, in the case of methionine-prochymosin, of more than 30% compared to, for example 10 to 20% for multiple alkali extractions.

Preferably the pH of the alkaline aqueous solution is from 9 to 11.5 most preferably from 10 to 11.

Preferably the dilution is from 10 fold to 50 fold. (That is a dilution into a total volume of from 10 to 50 volumes.

Preferably, in the solubilisation process of the invention, the insoluble protein is denatured in an aqueous solution comprising urea at a concentration of at least 7M or in a solution comprising guanidine hydrochloride at a concentration of at least 6M.

The pH is selected with reference to the protein to which the process is to be applied. In particular the pH is selected such that groups responsible for holding the protein in an unnatural conformation by means of intramolecular, or potentially in the case of a protein aggregate nonfunctional intermolecular, bonds or forces are dissociated such that, when the pH is reduced, the protein refolds in the native conformation. The groups responsible for holding the protein in an unnatural conformation may be ionisable groups, in which case the pH is preferably selected to be compatible with the pKa of the relevant ionisable group.

Studies in our laboratory have shown that intermolecular disulphide bonds exist in prochymosin aggregates produced in *E. coli* (Schoemaker *et al* (1984) submitted to PNAS). Native prochymosin is monomeric and contains three intramolecular disulphide bonds (Foltmann *et al* (1977) Proc. Natl. acad. Sci. U.S.A. *74* pp 2321—2324). Six thiol groups per molecule are therefore available to form intermolecular and intramolecular bonds within the protein aggregate. Consequently disulphide bonds must be broken and correctly reformed for denaturation/renaturation to successfully solubilise prochymosin. This may be achieved by using an alkaline aqueous solution of pH 10.7 (+0.5). The free thiol groups of cysteine have a pKa value of 10.46.

The term "insoluble" as used herein means in a form which, under substantially neutral conditions (for example pH in the range 5.5 to 8.5), is substantially insoluble or is in an insolubilised association with insoluble material produced on lysis of host organism cells. The insoluble product is either produced within the cells of the host organism in the form of insoluble relatively high molecular weight aggregates or may simply be associated with insoluble cell membrane material. The process permits the separation of solubilised protein from insoluble cellular debris.

Any suitable alkali may be used in the process, for exmaple an aqueous solution of an alkali metal hydroxide such as NaOH or KOH, an aqueous buffer, or an aqueous solution of an organic base such as triethylamine.

The insoluble protein may be a recombinant animal protein produced by a host organism. Examples of such proteins are immunoglobulin light and heavy chains polypeptides, foot and mouth disease antigens and thymosin and insulin proteins.

The host organism may be a naturally occurring organism or a mutated organism capable of producing an insoluble protein. Preferably, however, the host organism is an organism or the progeny of an organism which has been transformed using recombinant DNA techniques with a heterologous DNA sequence which codes for the production of a protein heterologous to the host organism and which is produced in an insoluble form. The host organism may be a eukaryotic organism such as a yeast or animal or plant cell. Preferred yeasts include *Saccharomyces cerevisiae* and *kluyveromyces.* In the alternative the host organism may be a bacterium such as *E. coli, B. subtilis, B. stearothermophilis* or *Pseudomonas.* Examples of specific host organism strains include *E. coli* HB101, *E. coli* X1776, *E. coli* X2882, *E. coli* PS410, *E. coli* RV308 and *E. coli* MRC1.

The host organism may be transformed with any suitable vector molecule including plasmids such as ColEI, pCRI, pBR322, RP4 and phage λ DNA or derivatives of any of these.

Prior to treatment with the process of the present invention the host cells may be subjected to an appropriate lysis or permeabilisation treatment to facilitate recovery of the product. For example, the host organism may be treated with an enzyme, for example a lysozome, or a mechanical cell destructing device to break down the cells.

The process of the invention may then be employed to solubilise the insolubilized product and the resulting solution may be separated from solid cell material such as insoluble cell membrane debris. Any suitable method including filtration or centrifugation may be used to separate solution containing the solubilised protein from the solid cell material.

The present invention is now illustrated by way of the following Examples:

Example 1

An experiment was conducted in which the solubilisation of methionine-prochymosin produced by *E. coli* cells transformed with vector pCT70 was achieved using denaturation with guanidine hydrochloride, followed published British patent application GB2100737A. by dilution into an alkaline solution. The

4

preparation of the transformed cell line is described in detail in published British patent application GB2100737A.

Frozen *E. coli* pCT70 cells grown under induced conditions were suspended in three times their own weight of 0.05 M Tris-HCl pH 8, 1 mM EDTA, 0.1 M NaCl, containing 23 μg/ml phenylmethylsulphonyl-fluoride (PMSF) and 130 μg/ml of lysozyme and the suspension was incubated at 4°C for 20 minutes. Sodium deoxycholate was added to a final concentration of 0.5% and 10 μg of DNA ase 1 (from bovine pancreas) was added per gram of *E. coli* starting material. The solution was incubated at 15°C for 30 minutes by which time the viscosity of the solution had decreased markedly. The extract, obtained as described above, was centrifuged for 45 minutes at 4°C and 10000 x g. At this stage effectively all the methionine-prochymosin product was in the pellet fraction in insolubilised form, presumably as a result of aggregation or binding to cellular debris. The pellet was washed in 3 volumes of 0.01 M Tris-HCl pH 8, 0.1 M NaCl, 1 mM EDTA at 4°C. The following manipulations were carried out a room temperature. After further centrifugation, as above, the supernatant solution was discarded and the pellet was dissolved in 3—5 volumes of buffer to a final concentration fo 6M guanidine HCl/0.05 M Tris pH 8, 1 mM EDTA, 0.1 M NaCl and allowed to stand for 30 minutes—2 hours. The mixture was diluted into 10—50 volumes of the above buffer at pH 10.7 lacking guanidine HCl. Dilution was effected by slow addition of the sample to the stirred diluent over a period of 10—30 minutes. The diluted mixture was readjusted to pH 10.7 by the addition of 1 M NaOH and allowed to stand for 10 minutes—2 hours. The pH was then adjusted to 8 by the addition of 1N HCl and the mixture allowed to stand for a further 30 minutes before centrifuging as above to remove precipitated proteins. The supernatant so produced contained soluble methionine-prochymosin which could be converted to catalytically active chymosin by acidification and neutralisation and purified as described in published British patent application GB2100737A. In a very similar experiment an 8M urea buffer was used in place of the 6M guanidine HCl buffer described above. The results were as described above.

### Example 2

An experiment was conducted in which the solubilisation of methionine-prochymosin produced by *E. coli* cells transformed with vector PCT70 was achieved using a method similar to that described in Example 1. The preparation of the transformed cell line is described in detail in published British patent application GB2100737A.

*E. coli*/pCT70 cell debris containing insoluble methionine-prochymosin was prepared and washed as described in Example 1 above and the following manipulations were carried out at room temperature. The washed pellets were suspended in a buffer containing 50 mM Tris HCl pH 8.0, 1 mM EDTA, 50 mM NaCl and 0.1 mM PMSF supplemented with 8 M urea (deionized). For every 10 g weight of starting material, 90 ml of buffer were used. After 1 hour, this solution was added slowly to a buffer containing 50 mM $KH_2PO_4$ pH 10.7 containing 1 mM EDTA and 50 mM NaCl and left for at least 30 minutes. Various dilutions were made in order to establish an optimum dilution range. This proved to be a dilution in alkali of between 10 and 50 fold. The pH was maintained at pH 10.7 for the period and then adjusted to pH 8. The product was activated to give active chymosin and the level of chymosin activity was assayed (Emtage, J. S., Angal, S., Doel, M. T. Harris, T. J. R. Jenkins, B., Lilley, G., and Low, P.A. (1983) Proc. Natl. Acad. Sci. USA *80*, 3671—3675). The results are shown in Table 1.

#### TABLE 1
Effect of dilution on the recovery of milk clotting activity.

| FOLD DILUTION | MILK CLOTTING ACTIVITY | | |
| --- | --- | --- | --- |
| UREA | ALKALI | OVERALL | (mg) |
| 1 | 10 | 10 | 0.04 |
| 1 | 25 | 25 | 0.10 |
| 1 | 50 | 50 | 0.05 |
| 5 | 10 | 50 | 0.10 |
| 5 | 25 | 125 | 0.09 |
| 5 | 50 | 250 | 1.23 |
| 10 | 10 | 100 | 1.97 |
| 10 | 25 | 250 | 1.56 |
| 10 | 50 | 500 | 0.09 |

**Claims for the Contracting States: AT BE FR DE LU NL SE CH LI**

1. A process for the production of a soluble native protein in which an insoluble form of the protein is produced by a host organism transformed with a vector including a gene coding for the protein, wherein the insoluble form of the protein is first denatured in an aqueous solution including a denaturant, and subsequently the resulting solution is diluted in an alkaline aqueous solution at a pH selected to promote dissociation of the group or groups of the protein involved in maintaining the conformation of the protein and the protein is renatured by reducing the pH of the solution below a pH effective to renature the protein, to produce the soluble native form of the protein, provided that the soluble native protein is not a chymosin precursor.

2. A process according to claim 2 wherein the pH of the alkaline aqueous solution is from 9 to 11.5.

3. A process according to claim 1 or 2 wherein the dilution is from 10 fold to 50 fold.

4. A process according to any one of the preceding claims wherein the insoluble protein is denatured in an aqueous solution comprising urea at a concentration of at least 7 M.

5. A process according to any one of claims 1 to 3 wherein the insoluble protein is denatured in an aqueous solution comprising guanidine hydrochloride at a concentration of at least 6 M.

**Claims for the Contracting State: IT**

1. A process for the production of a soluble native protein in which an insoluble form of the protein is produced by a host organism transformed with a vector including a gene coding for the protein, wherein the insoluble form of the protein is first denatured in an aqueous solution including a denaturant, and subsequently the resulting solution is diluted into an alkaline aqueous solution at a pH selected to promote dissociation of the group or groups of the protein involved in maintaining the conformation of the protein and the protein is renatured by reducing the pH of the solution below a pH effective to renature the protein, to produce the soluble native form of the protein.

2. A process according to claim 2 wherein the pH of the alkaline aqueous solution is from 9 to 11.5.

3. A process according to claim 1 or 2 wherein the dilution is from 10 fold to 50 fold.

4. A process according to any one of the preceding claims wherein the insoluble protein is denatured in an aqueous solution comprising urea at a concentration of at least 7 M.

5. A process according to any one of claims 1 to 3 wherein the insoluble protein is denatured in an aqueous solution comprising guanidine hydrochloride at a concentration of at least 6 M.

**Patentansprüche für die Vertragsstaaten: AT BE FR DE LU NL SE CH**

1. Verfahren zur Herstellung eines löslichen nativen Proteins, bei welchem eine unlösliche Form des Proteins durch einen Wirtsorganismus, der mit einem Vektor mit einer Genkodierung für das Protein transformiert worden ist, erzeugt wird, wobei die unlösliche Form des Proteins zuerst in einer wässerigen, ein Denaturierungsmittel enthaltenden Lösung denaturiert und anschließend die entstehende Lösung in einer alkalischen wässerigen Lösung bei einem pH-Wert verdünnt wird, der dahingehend ausgewählt wird, daß die Dissoziation einer Gruppe oder von Gruppen des Proteins, die mit der Aufrechterhaltung des Aufbaues des Proteins befaßt sind, begünstigt wird, und das Protein anschließend durch Herabsetzung des pH-Werts der Lösung auf einen Wert unterhalb eines zur Renaturierung des Proteins wirksamen Wert renaturiert wird, um die lösliche native Form des Proteins zu erzeugen, mit der Maßgabe, daß das lösliche, native Protein nicht eine Chymosin-Vorstufe ist.

2. Verfahren nach Anspruch 1, dadurch gekennzeichnet, daß der pH-Wert der alkalischen wässerigen Lösung 9 bis 11,5 beträgt.

3. Verfahren nach Anspruch 1 oder 2, dadurch gekennzeichnet, daß die Verdünnung 10- bis 50-fach ist.

4. Verfahren nach einem der vorhergehenden Ansprüche, dadurch gekennzeichnet, daß das unlösliche Protein in einer wässerigen Lösung von Harnstoff bei einer Konzentration von wenigstens 7 M denaturiert wird.

5. Verfahren nach einem der Ansprüche 1 bis 3, dadurch gekennzeichnet, daß das unlösliche Protein in einer wässerigen Lösung von Guanidinhydrochlorid mit einer Konzentration von wenigstens 6 M denaturiert wird.

**Patentansprüche für den Vertragsstaat: IT**

1. Verfahren zur Herstellung eines löslichen nativen Proteins, bei welchem eine unlösliche Form des Proteins durch einen Wirtsorganismus, der mit einem Vektor mit einer Genkodierung für das Protein transformiert worden ist, erzeugt wird, wobei die unlösliche Form des Proteins zuerst in einer wässerigen, ein Denaturierungsmittel enthaltenden Lösung denaturiert und anschließend die entstehende Lösung in einer alkalischen wässerigen Lösung bei einem pH-Wert verdünnt wird, der dahingehend ausgewählt wird, daß die Dissoziation einer Gruppe oder von Gruppen des Proteins, die mit der Aufrechterhaltung des Aufbaues des Proteins befaßt sind, begünstigt wird, und das Protein anschließend durch Herabsetzung des pH-Werts der Lösung auf einen Wert unterhalb eines zur Renaturierung des Proteins wirksamen Wert renaturiert wird, um die lösliche native Form des Proteins zu erzeugen.

2. Verfahren nach Anspruch 1, dadurch gekennzeichnet, daß der pH-Wert der alkalischen wässerigen Lösung 9 bis 11,5 beträgt.

3. Verfahren nach Anspruch 1 oder 2, dadurch gekennzeichnet, daß die Verdünnung 10- bis 50-fach ist.

4. Verfahren nach einem der vorhergehenden Ansprüche, dadurch gekennzeichnet, daß das unlösliche Protein in einer wässerigen Lösung von Harnstoff bei einer Konzentration von wenigstens 7 M denaturiert wird.

5. Verfahren nach einem der Ansprüche 1 bis 3, dadurch gekennzeichnet, daß das unlösliche Protein in einer wässerigen Lösung von Guanidinhydrochlorid mit einer Konzentration von wenigstens 6 M denaturiert wird.

## Revendications pour les Etats contractants: AT BE FR DE LU NL SE CH LI GB

1. Procédé de production d'une protéine native soluble, dans lequel une forme insoluble de la protéine est produit par un organisme hôte transformé à l'aide d'un vecteur comprenant un gène codant pour la protéine, dans lequel la forme insoluble de la protéine est tout d'abord dénaturée dans une solution aqueuse comprenant un agent dénaturant puis la solution résultante est diluée pour donner une solution aqueuse alcaline à un pH choisi pour favoriser la dissociation d'un ou de plusieurs groupes de la protéine impliqué(s) dans le maintien de la conformation de la protéine, et la protéine est remise dans son état naturél ("renaturée") par réduction du pH de la solution au dessous d'un pH capable de provoquer le retour efficace de la protéine dans son état naturel, afin de produire la forme native soluble de la protéine, à la condition que la protéine native soluble ne soit pas un précurseur de la chymosine.

2. Procédé selon la revendication 1, dans lequel le pH de la solution alcaline aqueuse est de 9 à 11,5.

3. Procédé selon la revendication 1 ou 2, dans lequel la dilution va de 10 fois à 50 fois.

4. Procédé selon l'une quelconque des revendications précédentes, dans lequel la protéine insoluble est dénaturée dans une solution aqueuse comprenant de l'urée présente en une concentration d'au moins 7 M.

5. Procédé selon l'une quelconque des revendications 1 à 3, dans lequel la protéine insoluble est dénaturée dans une solution aqueuse comprenant du chlorhydrate de guanidine, présent en une concentration d'aun moins 6 M.

## Revendications pour l'Etat contractant: IT

1. Procédé de production d'une protéine native soluble, dans lequel une forme insoluble de la protéine est produite par un organisme hôte transformé à l'aide d'un vecteur comprenant un gène codant pour la protéine, dans lequel la forme insoluble de la protéine est tout d'abord dénaturée dans une solution aqueuse comprenant un agent dénaturant puis la solution résultante est diluée pour donner une solution aqueuse alcaline à un pH choisi pour favoriser la dissociation d'un ou de plusieurs groupes de la protéine impliqué(s) dans le maintien de la conformation de la protéine, et la protéine est remise dans son état naturél ("renaturée") par réduction du pH de la solution au dessous d'un pH capable de provoquer le retour efficace de la protéine dans son état naturel, afin de produire la forme native soluble de la protéine.

2. Procédé selon la revendication 1, dans lequel le pH de la solution alcaline aqueuse est de 9 à 11,5.

3. Procédé selon la revendication 1 ou 2, dans lequel la dilution va de 10 fois à 50 fois.

4. Procédé selon l'une quelconque des revendications précédentes, dans lequel la protéine insoluble est dénaturée dans une solution aqueuse comprenant de l'urée présente en une concentration d'au moins 7 M.

5. Procédé selon l'une quelconque des revendications 1 à 3, dans lequel la protéine insoluble est dénaturée dans une solution aqueuse comprenant du chlorhydrate de guanidine, présent en une concentration d'aun moins 6 M.